# EUROPEAN PATENT APPLICATION

(11) **EP 3 257 431 A1**
(43) Date of publication of application: **20.12.2017**
(21) Application number: 16866483.7
(22) Date of filing: 21.11.2016
(51) Int. Cl.: A61B 1/06, G02B 23/24

(54) **ENDOSCOPE DEVICE**

(30) Priority: 19.11.2015 JP 2015227004
(71) Applicant: Olympus Corporation, Hachioji-shi Tokyo 192-8507 (JP)
(72) Inventor: NIWA, Hiroshi, Hachioji-shi Tokyo 192-8507 (JP); SATO, Minoru, Tokyo 192-8507 (JP)
(74) Representative: Gunzelmann, Rainer
(86) International application number: PCT/JP2016/084419
(87) International publication number: WO 2017/086483

(57) **Abstract**

An endoscope apparatus 1 includes: an endoscope connector 20; a first optical connector 51 extended from a plug section 41; first insertion openings 71 and 76 of a receptacle section 31 into which the first optical connector 51 is inserted; second insertion openings 72 and 77 of the receptacle section 31 into which a second optical connector 52 is inserted; a wrong insertion preventing member 74 that is partially located in the second insertion openings 72 and 77, and is moved, in conjunction with insertion of the first optical connector 51 into the first insertion openings 71 and 76, to a retracted position at which the second optical connector 52 is insertable into the second insertion openings 72 and 77; and a contact member 78 that comes into contact with the wrong insertion preventing member 74 when the first optical connector 51 is inserted into the second insertion openings 72 and 77.

## Description

### Technical Field

The present invention relates to an endoscope apparatus including a receptacle section to which an endoscope connector suitable for high-speed transmission is connected.

### Background Art

In a medical field, an endoscope that allows for observation of organs in a body cavity through insertion of an elongated insertion section into the body cavity is conventionally widely used.

To display an observation image of the organs in the body cavity, etc. on a monitor, an electronic endoscope in which a solid-state image pickup device such as a charge coupled device (CCD) is disposed in an image pickup section at a distal end or a rear end of the insertion section of the endoscope is used.

A signal outputted from the image pickup device provided in the electronic endoscope is converted into a video signal by an image processor that is provided separately from the electronic endoscope and an external camera, thereby being outputted to a monitor. Further, the electronic endoscope and the image processor that are provided separately from each other are connected to each other through an endoscope connector.

Such a connection configuration between a connector section of the electronic endoscope and the image processor is disclosed in, for example, Japanese Patent Application Laid-Open Publication No. 2013-158425. As for the connector section of the electronic endoscope and the endoscope disclosed in Japanese Patent Application Laid-Open Publication No. 2013-158425, a technology is disclosed that allows for smooth insertion of each plug into corresponding one of an electrical connection jack and an optical connection jack serving as an optical transmission plug of the image processor even if an impact load is applied to the electrical connection plug, the optical connection plug, and the like.

Incidentally, in the existing electronic endoscope, metallic contact pins are provided on each of the endoscope connector and the receptacle section of the image processor to perform electrical connection, and electrical signals such as image signals are transmitted.

In recent years, higher-speed transmission is demanded along with improvement of image quality of the endoscope, and an endoscope connector that includes an optical transmission plug for high-speed transmission as disclosed in Japanese Patent Application Laid-Open Publication No. 2013-158425 is developed.

In the case where a light guide plug for illumination is provided in the endoscope connector in addition to the optical transmission plug disclosed in Japanese Patent Application Laid-Open Publication No. 2013-158425, however, the light guide plug, the optical transmission plug, and the sockets of the receptacle section may be damaged if the plugs are wrongly inserted into the sockets.

Therefore, the present invention is made in consideration of the above-described circumstances, and an object of the present invention is to provide the endoscope apparatus that prevents wrong connection when the endoscope connector including the optical transmission plug and the light guide plug of the electronic endoscope is connected to the receptacle section of the image processor, and to accordingly prevent damage of the apparatuses.

### Disclosure of Invention

### Means for Solving the Problem

An endoscope apparatus according to an aspect of the present invention includes: an endoscope connector including a plug section; a male first optical connector extended from the plug section; a receptacle section to which the plug section is fitted; a first insertion opening that is provided in the receptacle section and into which the first optical connector is inserted; a second insertion opening that is provided in the receptacle section and into which a male second optical connector different from the first optical connector is inserted; a wrong insertion preventing member that is at least partially located in the second insertion opening in a state in which the first optical connector is not inserted into the first insertion opening, and is moved, in conjunction with insertion of the first optical connector into the first insertion opening, to a retracted position at which the second optical connector is insertable into the second insertion opening; and a contact member that comes into contact with the wrong insertion preventing member to maintain a state in which the wrong insertion preventing member is at least partially located in the second insertion opening when the first optical connector is inserted into the second insertion opening.

### Brief Description of the Drawings

Fig. 1 is a perspective view illustrating a configuration of an endoscope apparatus according to an aspect of the present invention;
Fig. 2 is a perspective view illustrating an endoscope connector according to the aspect of the present invention;
Fig. 3 is a perspective view illustrating a configuration of the endoscope connector as viewed from an angle different from an angle of Fig. 2 according to the aspect of the present invention;
Fig. 4 is a front view illustrating the configuration of the endoscope connector according to the aspect of the present invention;
Fig. 5 is a side view illustrating a configuration of a plug section of the endoscope connector according to the aspect of the present invention;
Fig. 6 is a perspective view illustrating a configuration of a receptacle section of an image processor according to the aspect of the present invention;
Fig. 7 is a front view illustrating the configuration of the receptacle section according to the aspect of the present invention;
Fig. 8 is a cross-sectional diagram illustrating the configuration of the receptacle section according to the aspect of the present invention;
Fig. 9 is a perspective view illustrating a configuration of a plate-like stopper member according to the aspect of the present invention;
Fig. 10 is a partial cross-sectional diagram to explain action when the endoscope connector is connected to the receptacle section, according to the aspect of the present invention;
Fig. 11 is a partial cross-sectional diagram to explain action when the endoscope connector is further inserted into the receptacle section from a state of Fig. 10, according to the aspect of the present invention;
Fig. 12 is a partial cross-sectional diagram to explain action when the endoscope connector is further inserted into the receptacle section from a state of Fig. 11, according to the aspect of the present invention;
Fig. 13 is a partial cross-sectional diagram illustrating a state where the endoscope connector has been connected to the receptacle section, according to the aspect of the present invention; and
Fig. 14 is a partial cross-sectional diagram to explain action that prevents the endoscope connector from being wrongly connected to the receptacle section, according to the aspect of the present invention.

### Best Mode for Carrying Out the Invention

Here, an endoscope apparatus that is the present invention is described with an example. Note that, in the following description, drawings based on respective embodiments are conceptual sketches and may not reflect actual relationship between a thickness and a width of each portion, actual proportion of the thickness of each portion, etc. Dimensional relationships and proportions may also differ from one drawing to another.

In addition, as for the endoscope apparatus in the following configuration description, a so-called rigid endoscope that is used for surgery and has a rigid insertion section is described as an example; however, the endoscope is not limited to the rigid endoscope. The endoscope apparatus in the following configuration description is a technology applicable to a so-called flexible endoscope in which an insertion section has flexibility for insertion into upper and lower digestive organs and the like of a living body.

An endoscope apparatus according to an aspect of the present invention is described below on the basis of the drawings.

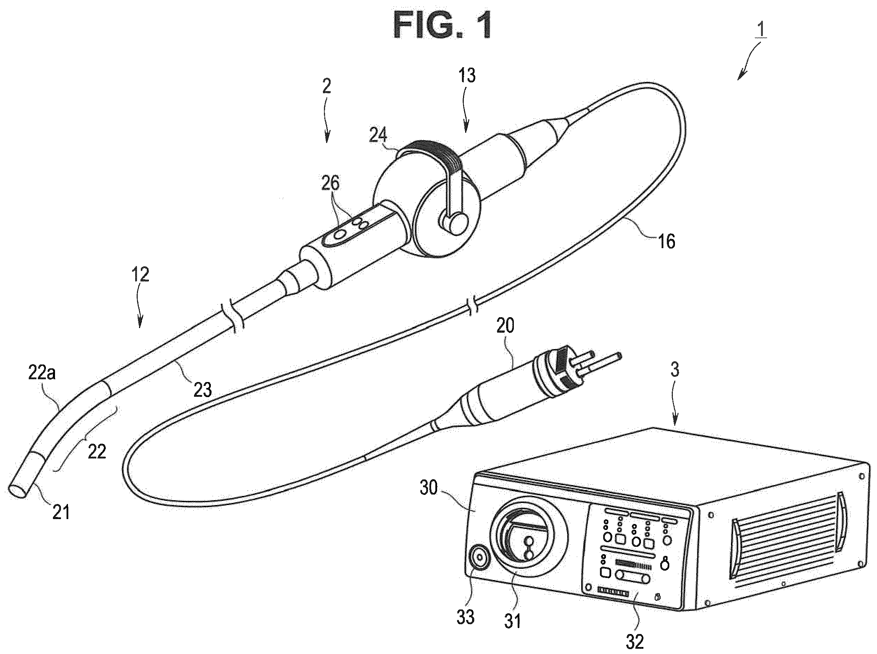

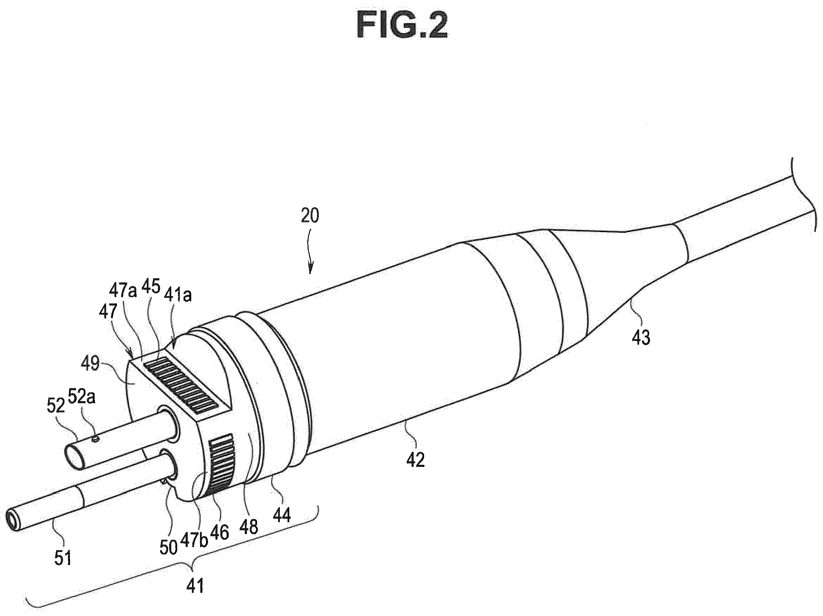

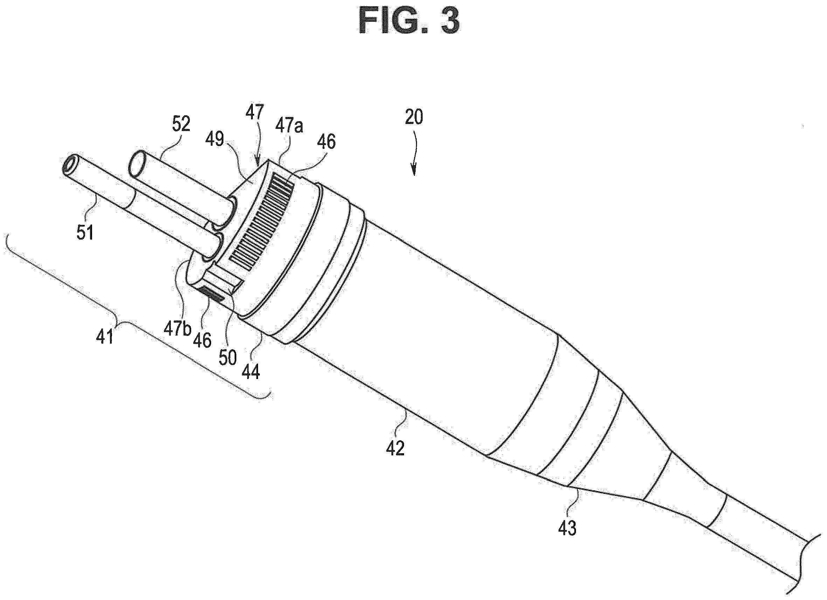

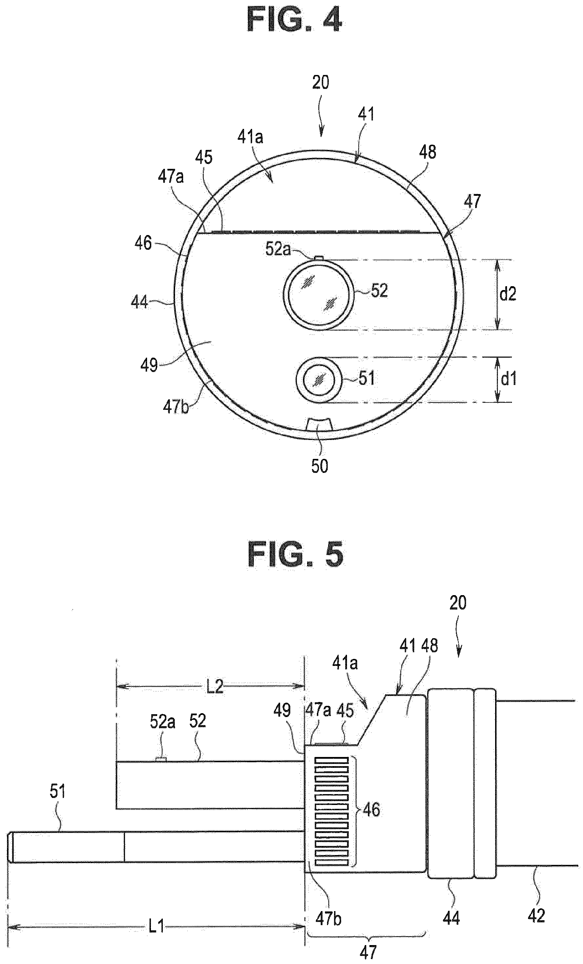

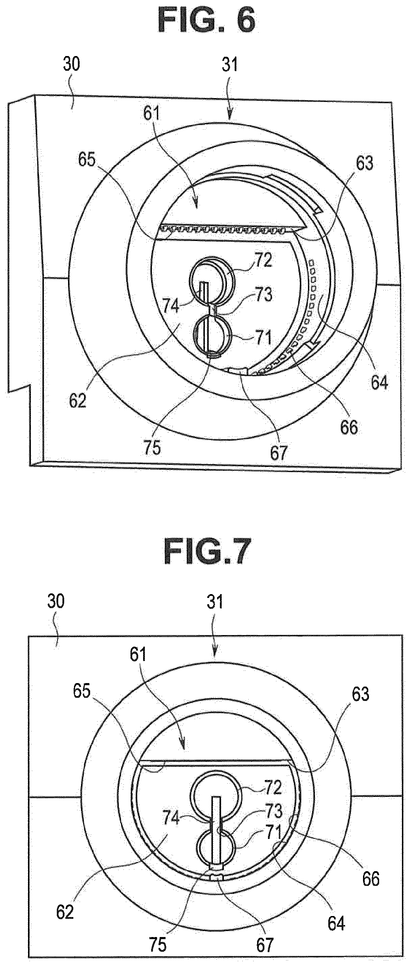

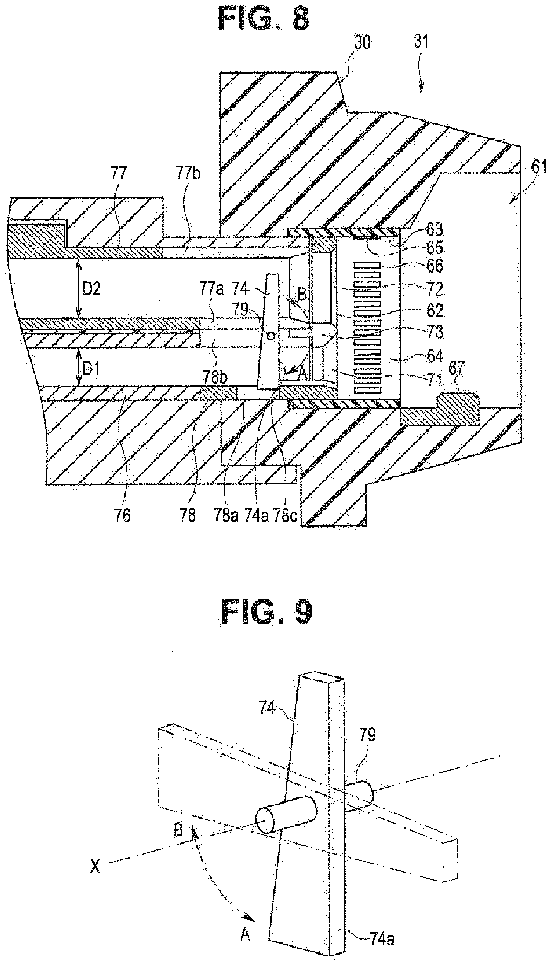

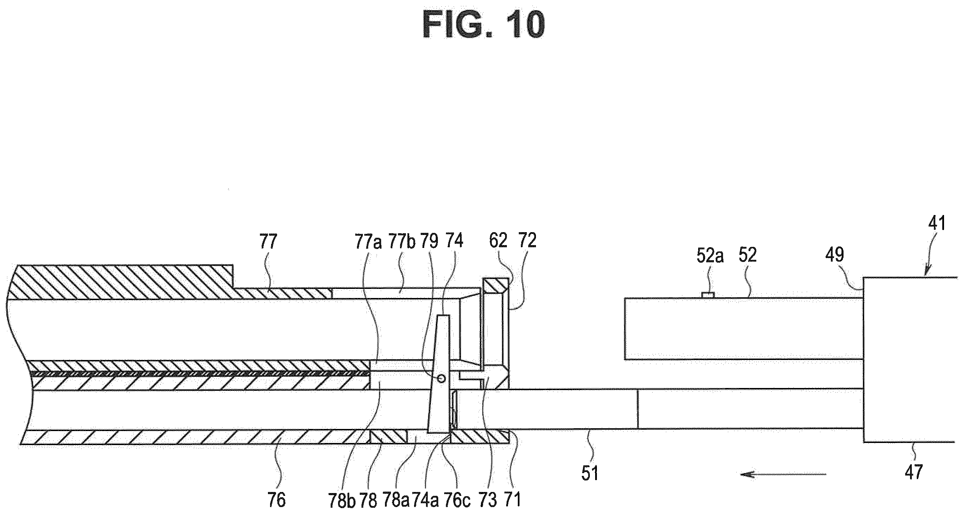

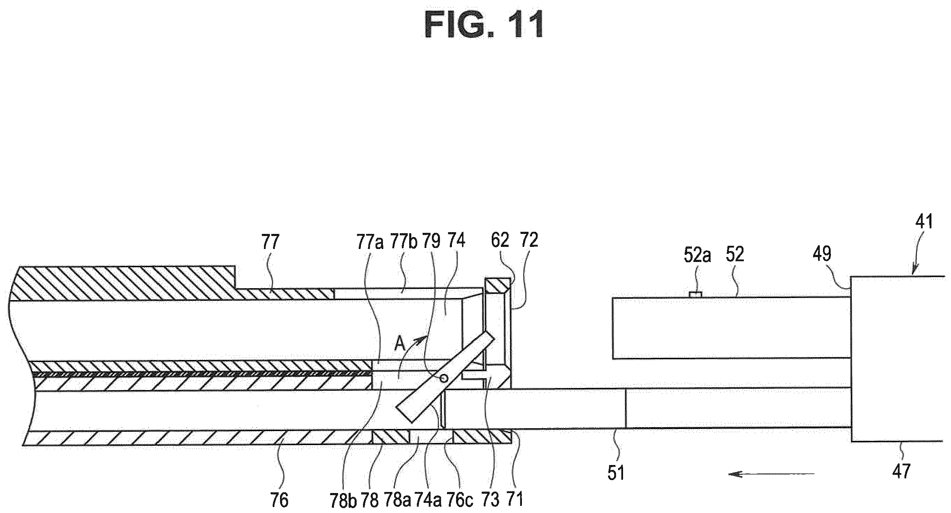

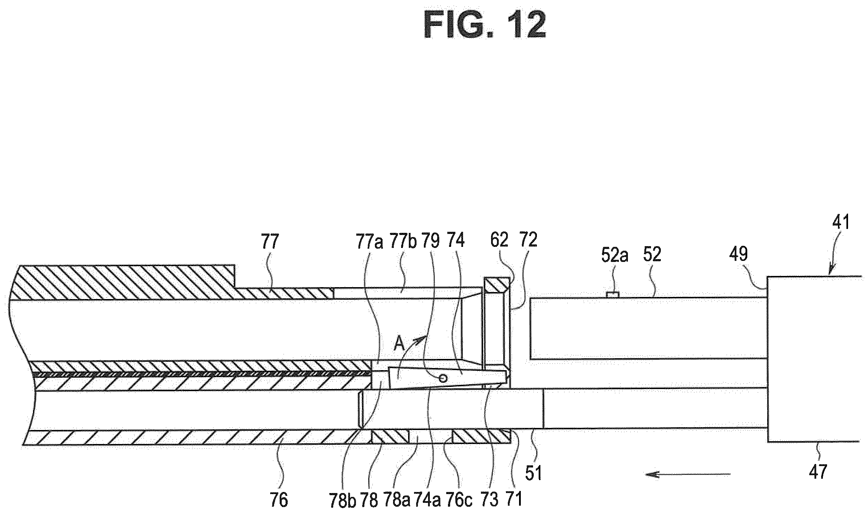

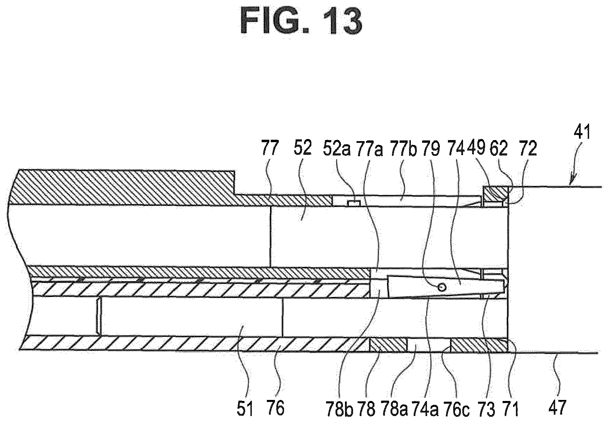

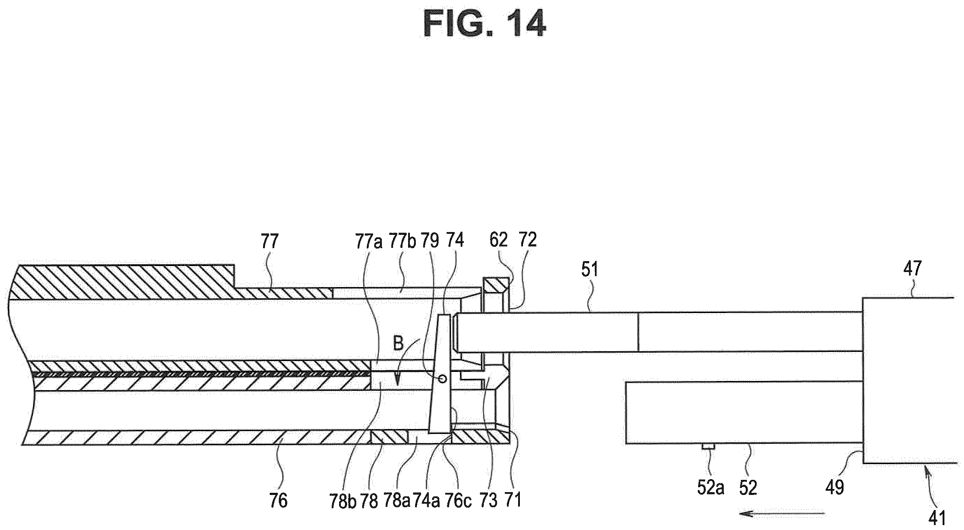

Note that Fig. 1 is a perspective view illustrating a configuration of the endoscope apparatus according to the aspect of the present invention, Fig. 2 is a perspective view illustrating an endoscope connector, Fig. 3 is a perspective view illustrating a configuration of the endoscope connector as viewed from an angle different from an angle of Fig. 2, Fig. 4 is a front view illustrating the configuration of the endoscope connector, Fig. 5 is a side view illustrating a configuration of a plug section of the endoscope connector, Fig. 6 is a perspective view illustrating a configuration of a receptacle section of an image processor, Fig. 7 is a front view illustrating the configuration of the receptacle section, Fig. 8 is a cross-sectional diagram illustrating the configuration of the receptacle section, Fig. 9 is a perspective view illustrating a configuration of a plate-like stopper member, Fig. 10 is a partial cross-sectional diagram to explain action when the endoscope connector is connected to the receptacle section, Fig. 11 is a partial cross-sectional diagram to explain action when the endoscope connector is further inserted into the receptacle section from a state of Fig. 10, Fig. 12 is a partial cross-sectional diagram to explain action when the endoscope connector is further inserted into the receptacle section from a state of Fig. 11, Fig. 13 is a partial cross-sectional diagram illustrating a state where the endoscope connector has been connected to the receptacle section, and Fig. 14 is a partial cross-sectional diagram to explain action that prevents the endoscope connector from being wrongly connected to the receptacle section.

As illustrated in Fig. 1, an endoscope apparatus 1 includes an endoscope 2 and an image processor (also referred to as an illumination light source built-in type video processor or a camera control unit) 3 that is an external apparatus also serving as a light source apparatus.

The endoscope 2 mainly includes: a long insertion section 12; an operation section 13 that is connected to a proximal end of the insertion section 12; and an endoscope connector 20 that is connected to the image processor 3.

Note that, in the endoscope 2, the operation section 13 and the endoscope connector 20 are connected to each other through a flexible cable 16 serving as a universal cord.

The insertion section 12 mainly includes a distal end portion 21, a flexibly-bendable bending portion 22, and a rigid pipe 23 that are connected in order from distal end side. The distal end portion 21 is configured of a metallic member such as stainless steel. The rigid pipe 23 is configured of a metal pipe such as stainless steel. The insertion section 12 is a section to be inserted mainly into an abdominal cavity, and includes an unillustrated communication cable, an unillustrated optical transmission fiber, an unillustrated light guide, and the like that are incorporated inside the insertion section 12. Note that the endoscope 2 according to the present embodiment is a so-called rigid endoscope for surgery in which the rigid insertion section 12 is inserted into an abdominal cavity of a living body.

The communication cable, the optical transmission fiber, the light guide, and the like are inserted into the flexible cable 16 through the operation section 13, and are extended up to the endoscope connector 20.

In the operation section 13, an angle lever 24 and various kinds of switches 26 are provided. The angle lever 24 is a bending operation member allowing for remote operation of the bending portion 22. The switches 26 are provided for operation of the image processor 3 and the like.

The angle lever 24 is bending operation means that allows for operation of the bending portion 22 of the insertion section 12 in vertical two directions. Note that two angle levers 24 may be provided to bend the bending portion 22 in vertical and lateral four directions.

An image pickup section using an unillustrated CCD sensor, an unillustrated CMOS sensor, or the like is incorporated in the distal end portion 21 of the insertion section 12. A communication cable for drive control and the optical transmission fiber for high-speed transmission of an image pickup signal are extended from the image pickup section.

A plurality of unillustrated bending pieces are provided inside the bending portion 22 of the insertion section 12. The plurality of bending pieces are rotated by an unillustrated bending operation wire, which causes the bending portion 22 of the insertion section 12 to bend. The unillustrated bending operation wire is pulled and relaxed by the angle lever 24. In addition, a bending rubber 22a that serves as an outer skin covering the plurality of bending pieces is provided in the bending portion 22.

The image processor 3 includes, on a front surface portion 30: a receptacle section 31 to which the endoscope connector 20 is connected; a panel section 32 for operation and state display; and a power supply switch 33. Note that an unillustrated illumination light source of the endoscope 2, such as a halogen lamp, is incorporated in the image processor 3.

Next, the configuration of the endoscope connector 20 is described in detail below.

As illustrated in Fig. 2 and Fig. 3, the endoscope connector 20 includes, in order from one end side, a plug section 41, a connector case 42, and a bend preventing portion 43 connected to the flexible cable 16. A flange portion 44 is provided between the plug section 41 and the connector case 42.

The plug section 41 includes: a fitting portion 47 in which electrical contacts (connector contacts) 45 and 46 configuring an electroconductive part are provided; and a connection portion 48 connected to the connector case 42.

In the plug section 41, the fitting portion 47 and the connection portion 48 are integrally formed. The plug section 41 is air-tightly connected to the connector case 42 through the metallic flange portion 44. Note that the flange portion 44 maintains the fitting state with the receptacle section 31 of the image processor 3.

The fitting portion 47 of the connector case 42 has a cutout part 41a that is obtained by cutting out a portion of a columnar shape similar to the connection portion 48, and includes a flat surface part 47a and a circumferential part 47b. The flat surface part 47a is parallel to a longitudinal axis (a center axis) direction of a cylindrical shape of the connector case 42, and the circumferential part 47b has a circumferential surface shape similar to a shape of the connection portion 48.

In other words, the fitting portion 47 has a so-called D-cut shape in which a portion of an outer columnar shape is cut to form the flat surface part 47a. Note that the fitting portion 47 is not limited to the D-cut shape, and has other shapes such as a simple outer columnar shape.

The connector contacts 45 and 46 serving as the electrical contacts are respectively provided on the flat surface part 47a and the circumferential part 47b of the fitting portion 47.

The plurality of connector contacts 45 and 46 each have a substantially rectangular surface shape. The plurality of connector contacts 45 are disposed side by side in a width direction on a surface of the flat surface part 47a, and the plurality of connector contacts 46 are disposed side by side in a circumferential direction on a circumferential surface of the circumferential part 47b.

A groove 50 is provided on a lower end part of an edge side of the circumferential part 47b of the fitting portion 47. The groove 50 causes the image processor 3 to recognize connection of the endoscope 2 and regulates a direction of the endoscope connector 20 around an axis when the endoscope connector 20 is connected to the receptacle section 31 of the image processor 3.

A front surface part 49 of the fitting portion 47 has an arc shape in which a portion of a circle is cut out by providing the flat surface part 47a. A substantially columnar light guide connector 51 and a substantially columnar optical connector 52 are extended from the front surface part 49. The light guide connector 51 is a first optical connector and serves as an illumination light guide plug. The optical connector 52 is a second optical connector and serves as an optical transmission connector (plug) for high-speed transmission. In other words, both of the light guide connector 51 and the optical connector 52 are male connectors (plugs) that are extended from the plug section 41 of the endoscope connector 20.

Note that the connector case 42 is formed in a substantially cylindrical shape having a diameter and a size that allow an operator to easily grip the connector case 42. The connector case 42 is a case body having the diameter that is slightly decreased toward the bend preventing portion 43 so as to be matched with the outer shape of the bend preventing portion 43.

Here, the light guide connector 51 and the optical connector 52 are described below.

As illustrated in Fig. 4, an outer diameter d1 of the light guide connector 51 in this case is set to be smaller than an outer diameter d2 of the optical connector 52 (d1<d2). Note that it is sufficient for the light guide connector 51 to have the outer diameter d1 equal to or smaller than the outer diameter d2 of the optical connector 52 (d1≤d2).

In addition, as illustrated in Fig. 5, a length L1 of the light guide connector 51 extended from the front surface part 49 of the fitting portion 47 is set to be larger than a length L2 of the optical connector 52 extended from the front surface part 49 (L1>L2).

Note that, in the light guide connector 51, an end part of the light guide for illumination light transmission disposed in the endoscope 2 is housed in the metallic cylinder, and an illumination optical system such as a lens is disposed at an end part of the metallic cylinder. The illumination optical system configures an end surface of the light guide connector 51.

Further, in the optical connector 52, an end part of the optical transmission fiber that is connected to the image pickup section incorporated in the distal end portion 21 of the insertion section 12 of the endoscope 2, is housed in the metallic cylinder. Note that the optical connector 52 is provided inside the metallic cylinder such that a core of the optical transmission fiber configures the end surface. A protrusion 52a is provided on an upper part on the outer circumference of the optical connector 52.

Next, the receptacle section 31 of the image processor 3 is described in detail below.

Electrical connection and optical transmission connection of the receptacle section 31 of the image processor 3 illustrated in Fig. 6 and Fig. 7 is performed when the plug section 41 of the endoscope connector 20 is inserted into the receptacle section 31.

The receptacle section 31 includes a fitting concave portion 61. The fitting concave portion 61 has an inner shape that is substantially coincident with an outer shape of the plug section 41 of the endoscope connector 20.

More specifically, the receptacle section 31 includes an unillustrated metallic mounting part that is disposed at a predetermined position of the fitting concave portion 61. The flange portion 44 of the plug section 41 of the endoscope connector 20 is fitted and fixed to the mounting part.

Further, the flange portion 44 of the plug section 41 and the mounting part of the receptacle section 31 are fitted and come into contact with each other, thereby connecting the ground of the endoscope 2 to the ground of the image processor 3 because both of the flange portion 44 and the mounting part are made of a metal. Note that it is sufficient for the mounting part of the receptacle section 31 to have a meal part for connection to the ground, and the mounting part of the receptacle section 31 may be fitted and fixed to the flange portion 44 of the plug section 41 through a non-metallic member.

The fitting concave portion 61 of the receptacle section 31 has the shape that is substantially coincident with the outer shape of the fitting portion 47 of the plug section 41. In other words, the fitting concave portion 61 includes a front surface part 62, a flat surface part 63, and an inner circumferential surface part 64. The front surface part 62 has a shape and a size that are substantially same as the shape and the size of the front surface part 49 of the fitting portion 47 of the plug section 41. The flat surface part 63 has a shape and a size that are substantially same as the shape and the size of the flat surface part 47a of the fitting portion 47. The inner circumferential surface part 64 has a shape and a size that are substantially same as the shape and the size of the circumferential part 47b of the fitting portion 47.

The fitting portion 47 of the endoscope connector 20 is inserted into the fitting concave portion 61 such that the front surface part 49 of the fitting portion 47 faces the front surface part 62 of the receptacle section 31.

At this time, the fitting portion 47 of the endoscope connector 20 is fitted to the fitting concave portion 61 of the receptacle section 31 when a direction around the longitudinal axis is aligned such that the flat surface part 47a and the circumferential part 47b of the fitting portion 47 respectively face the flat surface part 63 and the inner circumferential surface part 64 of the receptacle section 31.

Note that a plurality of receptacle contacts 65 serving as electrical contacts are provided at positions, on the flat surface part 63 of the receptacle section 31, respectively corresponding to the plurality of connector contacts 45 that are provided on the flat surface part 47a of the endoscope connector 20.

Moreover, a plurality of receptacle contacts 66 serving as electrical contacts are provided at positions, on the inner circumferential surface part 64 of the receptacle section 31, respectively corresponding to the plurality of connector contacts 46 that are provided on the circumferential part 47b of the endoscope connector 20.

As mentioned above, the connector contacts 45 and 46 respectively come into contact with the receptacle contacts 65 and 66 while the endoscope connector 20 is fitted and connected to the receptacle section 31, which results in electrical connection. This allows the endoscope 2 and the image processor 3 to exchange various kinds of electrical signals.

Note that the fitting concave portion 61 of the receptacle section 31 has a convex part 67 that projects upward from the lower end of the inner circumferential surface part 64. The convex part 67 engages with the groove 50 that is provided on the lower end part of the edge side of the circumferential part 47b of the fitting portion 47 of the endoscope connector 20 when the endoscope connector 20 is fitted and connected to the receptacle section 31.

As a result, the endoscope connector 20 is defined in the direction around the axis when being connected to the receptacle section 31 of the image processor 3, thereby causing the image processor 3 to recognize connection of the endoscope 2.

Incidentally, the light guide connector 51 and the optical connector 52 that are projected from the front surface part 49 of the endoscope connector 20 are respectively inserted into two openings 71 and 72 while the endoscope connector 20 is connected to the receptacle section 31. The two openings 71 and 72 are provided on the front surface part 62 of the receptacle section 31.

Note that the light guide connector 51 is inserted into the opening 71 that configures one of first insertion openings and the optical connector 52 is inserted into the opening 72 that configures one of second insertion openings, which causes the endoscope connector 20 to be connected to the receptacle section 31.

Here, configurations of female optical connectors (sockets) that are provided in the receptacle section 31 of the image processor 3 and into which the light guide connector 51 and the optical connector serving as the male optical connectors are respectively inserted and connected, are described in detail below.

As illustrated in Fig. 6 and Fig. 7, a slit 73 that has a predetermined width is so provided as to stride over the two openings 71 and 72 provided on the front surface part 62 of the receptacle section 31. The slit 73 makes the two openings 71 and 72 to communicate with each other.

A plate-like stopper member 74 serving as a wrong insertion preventing member is turnably disposed inside the slit 73. The plate-like stopper member 74 functions as a wrong insertion preventing key that causes the light guide connector 51 and the optical connector 52 to be inserted into the corresponding openings 71 and 72 without any mistake.

The plate-like stopper member 74 is so disposed as to stride over the openings 71 and 72. In addition, the plate-like stopper member 74 is so disposed as to be at least partially located in a region of the opening 72 while the light guide connector 51 and the optical connector 52 are not inserted into the respective openings 71 and 72.

As illustrated in Fig. 8, a light guide connector receiving pipe 76 and an optical connector receiving pipe 77 that respectively communicate with the two openings 71 and 72 provided on the front surface part 62 of the receptacle section 31 are provided inside the image processor 3. The light guide connector receiving pipe 76 configures one of the first insertion openings. The optical connector receiving pipe 77 configures one of the second insertion openings. The light guide connector receiving pipe 76 and the optical connector receiving pipe 77 are components of the female optical connectors.

Further, a contact pipe 78 is provided between the light guide connector receiving pipe 76 and the front surface part 62 in which the openings 71 and 72 are provided. The contact pipe 78 is a contact member that comes into contact with the plate-like stopper member 74 to stop turn of the plate-like stopper member 74.

Slits 78a and 78b each having a predetermined width are respectively provided on a lower part and an upper part of the contact pipe 78, and the plate-like stopper member 74 is so disposed as to be turnable in arrow AB direction in the drawing inside the slits 78a and 78b.

In the contact pipe 78, one end surface that forms the lower slit 78a and is orthogonal to the longitudinal direction includes a contact surface 78c. The contact surface 78c comes into contact with a lower end of the flat surface part 74a of the plate-like stopper member 74, thereby stopping turn of the plate-like stopper member 74 in an arrow B direction in the drawing, namely, in a counterclockwise direction in this example.

In addition, the contact pipe 78 includes a turning shaft 79 at a thick part that configures the upper slit 78b. The turning shaft 79 turnably supports the plate-like stopper member 74.

The light guide connector receiving pipe 76 is disposed at the position that communicates with the lower opening 71 through the contact pipe 78. An inner diameter D1 of the light guide connector receiving pipe 76 is set to be substantially equal to the outer diameter d1 of the light guide connector 51 (D1≈d1) or is set to a size that forms a slight clearance with the light guide connector 51. The light guide connector 51 is insertable into and removable from the light guide connector receiving pipe 76.

Note that an unillustrated connector section is provided in the light guide connector receiving pipe 76. The unillustrated connector section is connected to the light guide connector 51 and outputs illumination light emitted from the illumination light source.

The optical connector receiving pipe 77 is disposed at a position that communicates with the upper opening 72. An inner diameter D2 of the optical connector receiving pipe 77 is set to be substantially equal to the outer diameter d2 of the optical connector 52 (D2≈d2) or is set to a size that forms a slight clearance with the optical connector 52. The optical connector 52 is insertable into and removable from the optical connector receiving pipe 77.

In addition, slits 77a and 77b each having a predetermined width are respectively provided on a lower part and an upper part of the optical connector receiving pipe 77. The plate-like stopper member 74 is disposed in the lower slit 77a of the optical connector receiving pipe 77.

Note that the upper slit 77b of the optical connector receiving pipe 77 engages with the protrusion 52a provided on the optical connector 52, thereby rectilinearly guiding the insertion and removal of the optical connector 52.

An unillustrated connector section is provided in the optical connector receiving pipe 77. The unillustrated connector section is connected to the optical connector 52, thereby exchanging a laser beam for high-speed transmission that is suitable for transmission of, for example, a video signal.

The plate-like stopper member 74 is a plate body that has a thickness smaller than the predetermined width of each of the slits 73, 77a, and 78b, and has a trapezoidal side surface in which a bottom side is longer as illustrated in Fig. 9. Therefore, the bottom side of the plate-like stopper member 74 is naturally located on the lower side in the vertical direction due to the own weight by receiving gravity.

In other words, the plate-like stopper member 74 is erected such that the longitudinal direction is along the vertical direction in the state where the connectors 51 and 52 are not respectively inserted into the connector receiving pipes 76 and 77 that is the state where the endoscope connector 20 is not connected to the receptacle section 31.

Note that the plate-like stopper member 74 may have any other shape without being limited to the plate body having the trapezoidal side surface as long as the plate-like stopper member 74 is erected along the vertical direction by the own weight.

In the endoscope apparatus 1 having the above-described configuration, the operation when the endoscope connector 20 of the endoscope 2 is connected to the receptacle section 31 of the image processor 3 is described below. Note that, in the following, only components necessary for the operation description are illustrated.

When the endoscope connector 20 of the endoscope 2 is connected to the receptacle section 31 of the image processor 3, the light guide connector 51 is first inserted into the light guide connector receiving pipe 76 through the opening 71 that is provided on the front surface part 62 of the receptacle section 31 because the light guide connector 51 is longer in the length extended from the front surface part 49 of the fitting portion 47 than the optical connector 52, as illustrated in Fig. 10.

At this time, the end surface of the light guide connector 51 comes into contact with the lower end side (the exposed part on the opening 71 side) of the flat surface part 74a of the plate-like stopper member 74 that is erected by the own weight.

Then, as illustrated in Fig. 11, the plate-like stopper member 74 turns around the turning shaft 79 in the respective slits 73, 77a, and 78b (in this case, rotates in a clockwise direction illustrated by an arrow A in the drawing) in conjunction with insertion of the light guide connector 51 toward a deep part of the light guide connector receiving pipe 76.

Further, the light guide connector 51 is inserted into the deep part of the light guide connector receiving pipe 76, which causes the plate-like stopper member 74 to turn around the turning shaft 79. As a result, the plate-like stopper member 74 is housed in the respective slits 73, 77a, and 78b (is moved to a retracted position).

In this state, the optical connector 52 is not inserted into the optical connector receiving pipe 77 through the opening 72 provided on the front surface part 62 of the receptacle section 31. In other words, the optical connector 52 is not inserted into the optical connector receiving pipe 77 until the plate-like stopper member 74 is housed in the respective slits 73, 77a, and 78b (is moved to the retracted position).

To realize the configuration, the length of the bottom side of the plate-like stopper member 74 is set to be smaller than the height of the slit 73 and the total height of the two slits 77a and 78b, and the length L2 of the optical connector 52 extended from the front surface part 49 of the fitting portion 47 is set depending on the length L1 of the light guide connector 51 extended from the front surface part 49 of the fitting portion 47.

Then, the light guide connector 51 is further inserted into the deep part of the light guide connector receiving pipe 76 and the optical connector 52 is accordingly inserted into the optical connector receiving pipe 77 through the opening 72 provided on the front surface part 62 of the receptacle section 31 while the plate-like stopper member 74 is housed in the respective slits 73, 77a, and 78b.

At this time, the protrusion 52a of the optical connector 52 engages with the upper slit 77b of the optical connector receiving pipe 77 and the convex part 67 of the receptacle section 31 engages with the groove 50 of the fitting portion 47, which causes the endoscope connector 20 to be rectilinearly guided in the connection to the receptacle section 31.

Further, as illustrated in Fig. 12, the front surface part 49 of the fitting portion 47 is pushed until coming into contact with the front surface part 62 of the receptacle section 31. As a result, the light guide connector 51 of the endoscope connector 20 is connected to the connector section in the light guide connector receiving pipe 76 and the optical connector 52 is connected to the connector section in the optical connector receiving pipe 77.

As a result, the endoscope connector 20 of the endoscope 2 is fitted and connected to the receptacle section 31 of the image processor 3. Note that, in this state, the flange portion 44 is fitted and fixed to the mounting part of the receptacle section 31, and the connector contacts 45 and 46 of the fitting portion 47 and the receptacle contacts 65 and 66 of the receptacle section 31 are respectively in contact with and electrically connected to one another.

Note that if the user tries to insert the light guide connector 51 into the optical connector receiving pipe 77 that normally receives the optical connector 52, through the opening 72 provided in the front surface part 62 of the receptacle section 31 in a wrong vertical direction of the endoscope connector 20, the endoscope connector 20 is maintained in the state in which the end surface of the light guide connector 51 is in contact with the flat surface part 74a of the plate-like stopper member 74 to prevent further insertion of the light guide connector 51, as illustrated in Fig. 13.

In other words, when the end surface of the light guide connector 51 is in contact with the flat surface part 74a (the exposed part on the opening 72 side) of the plate-like stopper member 74 on the optical connector receiving pipe 77 side, the plate-like stopper member 74 tries to turn in the counterclockwise direction (in an arrow B direction in the drawing) around the turning shaft 79.

At this time, the lower end of the flat surface part 74a of the plate-like stopper member 74 abuts on the contact surface 78c of the contact pipe 78 to restrict the turn of the plate-like stopper member 74. As a result, the plate-like stopper member 74 does not move from the erected state along the vertical direction and the state where the plate-like stopper member 74 is at least partially located in the region of the opening 72 is maintained.

Accordingly, the light guide connector 51 is caught by the plate-like stopper member 74 and is not inserted any more, which makes it possible to prevent further insertion of the light guide connector 51 toward the deep part of the optical connector receiving pipe 77.

As described above, the endoscope apparatus 1 according to the present embodiment has the configuration in which the two male optical connectors (the plugs) of the endoscope connector 20 are easily and concurrently connected to the two female optical connectors (the sockets) of the image processor 3 through one operation to connect, to the receptacle section 31 of the image processor 3, the endoscope connector 20 of the endoscope 2 that includes, in addition to the light guide connector 51, the optical connector 52 serving as the optical transmission plug for higher-speed transmission along with improvement of image quality of the endoscope.

Further, the endoscope apparatus 1 has the configuration to prevent the male optical connectors (the plugs) from being inserted into the incorrect female optical connectors (the sockets) even if the endoscope connector 20 of the endoscope 2 is tried to be connected to the receptacle section 31 of the image processor 3 in the wrong direction. Therefore, it is possible to prevent wrong connection and to prevent damage of the male optical connectors (the plugs) and the female optical connectors (the sockets) caused by the wrong connection.

Therefore, the endoscope apparatus 1 according to the present embodiment makes it possible to prevent wrong connection when the endoscope connector 20 including the optical transmission plug and the light guide plug of the endoscope 2 is connected to the receptacle section 31 of the image processor 3, and to accordingly prevent damage of the apparatuses.

Note that the image processor 3 may preferably have connection compatibility with an existing endoscope connector in which the receptacle section 31 includes the light guide connector 51 and the connector contacts 45 and 46 without the optical connector 52 as described in the present embodiment, to allow for usage of an existing endoscope owned by the user.

In other words, the endoscope apparatus 1 may preferably have a configuration in which various kinds of electrical signals including an image pickup signal are exchanged through electrical connection between the receptacle contacts 65 and 66 provided in the receptacle section 31 of the image processor 3 and the connector contacts 45 and 46 provided in the endoscope connector (20) of the existing endoscope (2) owned by the user.

As mentioned above, the configuration in which the endoscope connector 20 of the endoscope 2 according to the present embodiment and the endoscope connector of the existing endoscope are connectable to the receptacle section 31 of the image processor 3 makes it possible to eliminate the need for the user to own some model-dependent image processors (3), thereby contributing to cost reduction for the user such as a hospital.

Note that even if the light guide connector 51 provided on the endoscope connector (20) of the existing endoscope (2) is tried to be inserted into the optical connector receiving pipe 77 that normally receives the optical connector 52, through the opening 72 provided on the front surface part 62 of the receptacle section 31, the end surface of the light guide connector 51 is in contact with the flat surface part 74a of the plate-like stopper member 74 to prevent further insertion of the light guide connector 51, as with the above description. This makes it possible to prevent the wrong connection and to accordingly prevent damage of the male optical connectors (the plugs) and the female optical connectors (the sockets) caused by the wrong connection in the image processor 3.

The invention described in the above-described embodiment is not limited to the embodiment and the modifications, and may be variously modified without departing from the scope of the present invention in execution stage. Further, the above-described embodiment encompasses inventions in various stages, and various inventions may be extracted through appropriate combination of the plurality of disclosed components.

For example, even if some components are deleted from all the components described in the above-described embodiment, a configuration with the components deleted may be extracted as the invention if such a configuration can solve a problem to be solved by the invention and provides the described effects of the invention.

The present invention makes it possible to provide the endoscope apparatus that prevents wrong connection of the endoscope connector including the optical transmission plug and the light guide plug of the electronic endoscope to the receptacle section of the image processor, thereby preventing damage of the apparatuses.

The present application is based upon and claims the benefit of priority of Japanese Patent Application No. 2015-227004 filed in Japan on November 19, 2015, the disclosed contents of which are incorporated in the specification and the claims of the present application by reference.

## Claims

1. An endoscope apparatus, comprising:
an endoscope connector including a plug section;
a male first optical connector extended from the plug section;
a receptacle section to which the plug section is fitted;
a first insertion opening that is provided in the receptacle section and into which the first optical connector is inserted;
a second insertion opening that is provided in the receptacle section and into which a male second optical connector different from the first optical connector is inserted;
a wrong insertion preventing member that is at least partially located in the second insertion opening in a state in which the first optical connector is not inserted into the first insertion opening, and is moved, in conjunction with insertion of the first optical connector into the first insertion opening, to a retracted position at which the second optical connector is insertable into the second insertion opening; and
a contact member that comes into contact with the wrong insertion preventing member to maintain a state in which the wrong insertion preventing member is at least partially located in the second insertion opening when the first optical connector is inserted into the second insertion opening.

2. The endoscope apparatus according to claim 1, wherein
the first optical connector is a light guide connector for illumination light, and
the light guide connector is inserted into the first insertion opening to cause illumination light illuminating a subject to enter the light guide connector.

3. The endoscope apparatus according to claim 1 or 2, wherein
the second optical connector is an optical transmission connector extended from the plug section, and
the optical transmission connector is inserted into the second insertion opening to cause a laser beam for high-speed transmission to be exchanged through the optical transmission connector.

4. The endoscope apparatus according to any one of claims 1 to 3, wherein the first optical connector is longer in a length extended from the plug section than the second optical connector.

5. The endoscope apparatus according to any one of claims 1 to 4, wherein the first optical connector has an outer diameter that is equal to or smaller than an inner diameter of the second insertion opening.

6. The endoscope apparatus according to any one of claims 1 to 5, wherein the wrong insertion preventing member is turnably disposed to stride over the first insertion opening and the second insertion opening, and turns to move to the retracted position by coming into contact with the first optical connector inserted into the first insertion opening.

7. The endoscope apparatus according to claim 6, wherein the wrong insertion preventing member turns, by own weight, to a position at which the wrong insertion preventing member is at least partially located in the second insertion opening while the first optical connector and/or the second optical connector is not inserted into the first insertion opening and/or the second insertion opening.
